(19) Europäisches Patentamt
European Patent Office
Office européen des brevets

(11) Numéro de publication : **0 125 152**
**B1**

(12) # FASCICULE DE BREVET EUROPÉEN

(45) Date de publication du fascicule du brevet : **30.12.86**

(51) Int. Cl.[4] : **C 08 B 37/14**, A 61 K 31/72

(21) Numéro de dépôt : **84400438.2**

(22) Date de dépôt : **05.03.84**

(54) **Nouveaux sulfates de xylanes, leur procédé de préparation et leur activité anti-thrombotique et hypolipémiante.**

(30) Priorité : **24.03.83 FR 8305170**

(43) Date de publication de la demande :
**14.11.84 Bulletin 84/46**

(45) Mention de la délivrance du brevet :
**30.12.86 Bulletin 86/52**

(84) Etats contractants désignés :
**AT BE CH DE FR GB IT LI LU NL SE**

(56) Documents cités :
**CH-A- 290 145**
**FR-A- 1 050 576**
**PATENTS ABSTRACTS OF JAPAN, vol. 2, no. 46, 28 mars 1978, page 5059 C 77**
**CHEMICAL ABSTRACTS, vol. 41, no. 18, 20 septembre 1947, colonnes 5983i-5984c, Columbus, Ohio, US; E. HUSEMANN et al.: "Blood anticoagulants"**
**CHEMICAL ABSTRACTS, vol. 80, no. 15, 15 avril 1974, page 452, no. 83526b, Columbus, Ohio, US**
**CHEMICAL ABSTRACTS, vol. 83, no. 2, 14 juillet 1975, page 136, no. 12705k, Columbus, Ohio, US**

(73) Titulaire : **SANOFI, société anonyme**
**40, Avenue George V**
**F-75008 Paris (FR)**

**ANIC S.p.A.**
**Piazza Ruggiero Settimo, 55**
**I-90139 Palermo (IT)**

(72) Inventeur : **Bayol, Alain**
**5 Rue de la Sardane**
**F-31170 Tournefeuille (FR)**
Inventeur : **Lansen, Jacqueline**
**27 Allée du Bon Accueil**
**F-34100 Montpellier (FR)**
Inventeur : **Maffrand, Jean-Pierre**
**5 Rue du Corps-Franc Pommiès**
**F-31120 Portet/Garonne (FR)**
Inventeur : **Pereillo, Jean-Marie**
**9 Avenue des Mimosas**
**F-31120 Portet/Garonne (FR)**
Inventeur : **Vallee, Eric**
**253 Chemin du Ramelet-Moundi**
**F-31170 Tournefeuille (FR)**

(74) Mandataire : **Polus, Camille et al**
**c/o Cabinet Lavoix 2, Place d'Estienne d'Orves**
**F-75441 Paris Cedex 09 (FR)**

**EP 0 125 152 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention est relative à de nouveaux sulfates de xylanes, à leur procédé de préparation et à leur application en médecine humaine et vétérinaire.

Il est classique, lorsqu'on désire obtenir en thérapeutique une action anti-coagulante et anti-thrombotique, d'utiliser généralement l'héparine. Cette dernière, qui est une substance d'extraction animale appartenant à la classe des mucopolysaccharides acides est ainsi administrée en clinique, comme agent anti-coagulant, par exemple en hémodialyse rénale, et comme agent antithrombotique dans la prévention et le traitement des thromboses veineuses profondes et dans le traitement des thromboses artérielles et des embolies pulmonaires. Cependant, cette thérapeutique présente d'assez sérieux inconvénients, liés d'une part aux risques d'hémorragie qu'elle provoque, et d'autre part au fait qu'on observe fréquemment, lors de son administration, des thrombopénies accompagnées dans certains cas de complications thrombo-emboliques et éventuellement associées à des thromboses, ainsi qu'un certain nombre d'autres effets secondaires (chocs, alopécies, ostéoporoses).

On connaît, par ailleurs, un autre polysaccharide sulfaté, appelé dans la littérature SP 54 ou PZ 68, qui présente une efficacité clinique comparable à l'héparine.

Ce composé, qui est obtenu par hémisynthèse à partir du bois du hêtre, présente en outre la caractéristique d'être d'un coût très inférieur à celui de l'héparine. Il se présente sous la forme d'un mélange de sulfates de xylanes de différents poids moléculaires. Ce produit est cependant mal défini et on trouve à son sujet dans la littérature différentes caractéristiques analytiques.

Ainsi, VINAZZER et al. reportent successivement un poids moléculaire moyen de 2 000 (Thromb. Res, 1980, *20*, 57-68) puis de 3 000 (thromb. Res, 1982, *27*, 341-352). C. O. ESQUIVEL et al. (Thromb Res, 1982, *28*, 389-399) décrivent un poids moléculaire moyen de 4 000 ; enfin C. SORIA et al. (Thromb Res 1980, *19*, 455-463) signalent un poids moléculaire moyen de 6 000.

Cette disparité dans les poids moléculaires moyens, est sans doute due aux méthodes d'analyse et aux standards utilisés ainsi qu'à l'absence de précision sur la nature (apparente, en poids, en nombre) de la masse molaire publiée.

La demanderesse a trouvé que ce produit, qui répond à la formule statistique développée suivante :

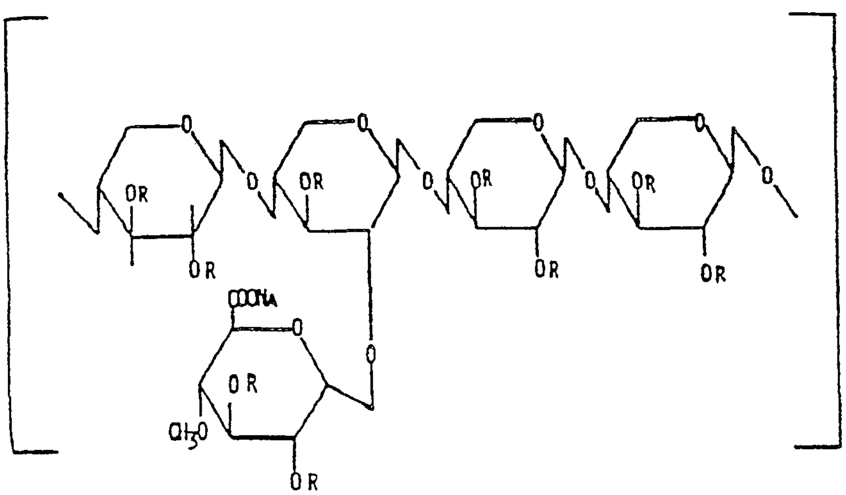

(I)

dans laquelle R représente le groupe —$SO_3Na$ ou l'hydrogène dans des proportions telles que le degré de sulfatation (D. S.) soit de 1,81 est défini par les caractéres analytiques suivants :

Ma = (masses moléculaires moyennes apparentes) : 6 000

$\overline{Mn}$ = (masses moléculaires moyennes en nombre) : 5 400

$\overline{Mw}$ = (masses moléculaires moyennes en poids) : 7 000

Par degré de sulfatation, on désigne le nombre de groupes —$SO_3Na$ par motif monomère de xylose.

Ces déterminations de masses moléculaires ont été confirmées par une mesure de la masse molaire par dosage de formol après oxydation des extrémités réductrices : M = 6 300.

Le produit SP 54 est encore défini par les caractéristiques suivantes :

Dispersion : D = $\overline{Mw/Mn}$ = 1,29

% d'acides uroniques : 5,8

% de pentoses : 35,5

La présente invention concerne de nouvelles fractions de sulfates de xylanes, répondant encore à la formule (I) mais définies par une masse molaire apparente comprise entre 7 000 à 12 000 et par les autres caractéristiques suivantes :

le degré de sulfatation est compris entre 1,5 et 2,0

le pourcentage en acide uronique est compris entre 0 et 10

le pourcentage en pentose est compris entre 30 et 40,

les nouvelles fractions conservant une efficacité clinique comparable à celle de l'héparine.

Les caractères analytiques ci-dessus ont été déterminés d'après les méthodes ci-après :

1. Détermination de la masse moléculaire : Elle est réalisée par chromatographie d'exclusion à travers deux colonnes de silice poreuse placées en série. L'appareil utilisé est celui de HEWLETT PACKARD 1081 B équipé d'un réfractomètre WATERS R 401 et d'un injecteur HP 79841 A. L'ensemble est thermostaté à 30 °C. La phase mobile est préparée selon BARTH H.G. et SMITH D.A. (J. Chromatogr, 1981, *206*, 410-415) et modifiée en ajoutant du polyéthylène glycol pour éviter les interactions de type pont hydrogène. Les masses moléculaires moyennes en nombre ($\overline{Mn}$), en poids ($\overline{Mw}$) et apparente (Ma) sont calculées à partir d'un étalonnage réalisé à l'aide d'oligosaccharides neutres et de Dextrans.

2. Masse moléculaire moyenne en nombre par libération de formol

Xylane : La détermination de la masse moléculaire absolue moyenne en nombre est réalisée par la méthode de VASKOVSKI V.E. et ISAY S.V. (Anal Biochem, 1969, *30*, 25-31) après réduction par le borohydrure de sodium.

Polysulfate de xylane : La détermination absolue de la masse moléculaire moyenne en nombre est réalisée comme pour les xylanes mais après désulfatation du produit.

3. Le degré de dispersion (D) est défini par le rapport $\overline{Mw/Mn}$

4. Dosage des acides uroniques : La méthode employée est celle au carbazole de BITTER T. et MUIR H.M. (Anal Biochem, 1962, 4, 330-334).

5. Microdosage du soufre : Après minéralisation en fiole de SHÖNIGER W. (Mikrochim Acta, 1956, *1*, 869-876), le soufre est dosé conductrimétriquement par perchlorate de baryum.

Le degré de sulfatation (D.S.) est défini par 5 fois le rapport S (moles)/C (moles).

6. Dosage des pentoses : La méthode employée est celle à l'orcinol et au chlorure ferrique en milieu chlorhydrique (MEJBAUM W. - Z. Physiol. Chem, 1979, *258*, 117) en prenant le xylose comme étalon.

L'invention comprend également un procédé de préparation des fractions de l'invention de la formule I ci-dessus, caractérisée en ce que le produit SP 54 est soumis directement à des fractionnements qui peuvent être obtenus, soit par chromatographie sur colonne, soit par ultra-filtration.

Ce fractionnement peut être effectué également sur les xylanes obtenus après désulfatation du SP 54.

Selon cette variante, le sel de pyridinium du SP 54 est soumis à une désulfatation par traitement avec du diméthyl-sulfoxyde additionné de 3 % à 10 % d'eau, à des températures comprises entre 50 °C et 100 °C.

Le xylane obtenu, qui répond à la formule développée suivante :

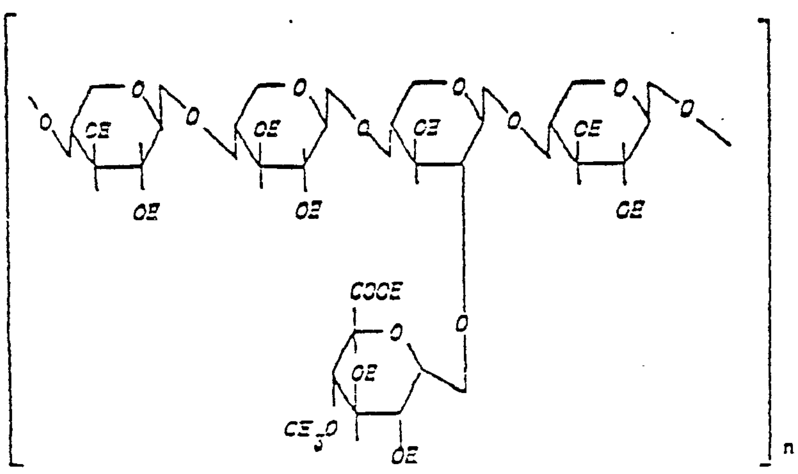

II

est défini par les caractéristiques ci-après :

Ma = 3 000 - $\overline{Mn}$ = 1 950 - $\overline{Mw}$ = 3 500 - D = 1,81

Masse moléculaire (dosage chimique) M = 2 000

Degré de sulfatation (D.S.) = 0

Pourcentage d'acides uroniques : 16,5 % en poids (exprimé en acide glucuronique)

Pourcentage de pentoses : 79,5 % en poids (exprimé en xylose monomère).

Le xylane est alors fractionné par solubilisation et/ou par chromatographie sur colonne, ou ultra-

filtration. On choisit ensuite les fractions recherchées qui, par sulfatation, conduisent aux composés de l'invention.

Cette sulfatation est effectuée soit par l'acide chlorosulfonique en présence de pyridine et éventuellement de diméthylformamide (D.M.F.), soit par le complexe trioxyde de soufre-triméthylamine dans le diméthylformamide.

Les exemples non limitatifs suivant illustrent l'invention.

La matière de départ SP 54 utilisée dans ces exemples est celle dont les caractéristiques analytiques ont été définies ci-dessus, et les caractéristiques analytiques des produits finaux ont été déterminées selon les mêmes méthodes.

Exemple 1

Désulfatation du polysulfate de xylane (SP 54)

Une solution de 60 g de polysulfate de xylane SP 54 (sel de sodium) dans 80 ml d'eau distillée est éluée avec de l'eau distillée sur une colonne de 500 ml de résine échangeuse de cations (IRN 77 - Forme $H^+$). Les éluats acides sont directement recueillis dans un récipient contenant 32 ml de pyridine et placés dans un bain de glace.

La solution obtenue est lyophilisée. Le sel de pyridinium du polysulfate de xylane est récupéré sous forme de poudre blanche : m = 68 g. Une solution de 36 g de ce sel de pyridinium dans 100 ml de diméthylsulfoxyde à 5 % d'eau est chauffée pendant 4 heures à 80 °C. Après refroidissement, le milieu réactionnel est versé dans 600 ml de méthanol. Le précipité formé est recueilli par filtration, lavé avec de l'éthanol, puis redissous dans 100 ml d'eau distillée. Le pH de cette solution est ajusté à 7,0 par addition de soude N. Puis cette solution est dessalée en trois opérations sur une colonne préparative de gel Séphadex G 15 (longueur 45 cm, diamètre 10 cm), éluée avec de l'eau distillée. Les fractions de têtes qui contiennent le polymère désulfaté, exempt de sulfate minéral sont recueillies puis lyophilisées. On recueille 11,3 g de xylane désulfaté.

Caractéristiques analytiques

$\overline{Mn}$ = 1 950 $\overline{Mw}$ = 3 500 D = 1,81 Ma = 3 000
Masse moléculaire (dosage chimique) = 2 000
Degré de sulfatation = nul
Pourcentage d'acides uroniques = 16,5 % en poids (exprimé en acide glucuronique).
Pourcentage de pentoses = 79,5 % en poids (exprimé en xylose monomère)

Exemple 2

Fractionnement du Xylane par solubilisation puis sulfatation.

Fractionnement du xylane

On ajoute en petites portions, sous agitation magnétique, 150 g de xylane (exemple 1) dans 1 400 ml d'eau distillée maintenue à 50 °C.

On laisse revenir la suspension à température ambiante.

1. la fraction de xylane insoluble est séparée par centrifugation puis remise en suspension dans l'eau et lyophilisée.

Poids 44,1 g : rendement en poids : 29,4 %.

Pourcentage d'acides uroniques : 15,76 % en poids (exprimé en acide glucuronique).

2. La fraction soluble du xylane (surnageant) est lyophilisée à son tour. On récupère 85,5 g de poudre de couleur crème. Rendement en poids : 57,7 %. Pourcentage d'acides uroniques : 17,02 % en poids (exprimé en acide glucoronique).

Sulfatation de la fraction de xylane insoluble dans l'eau à 10 % poids/volume.

Dans un tricol de 500 ml, on dissout sous atmosphère d'azote et agitation magnétique 12 g du xylane ci-dessus dans un mélange de 65 ml de pyridine anhydre et 65 ml de diméthylformamide anhydre.

On ajoute goutte à goutte dans la solution maintenue à 0 °C, 28,3 g (16,2 ml) d'acide chlorosulfonique. Après la fin de l'addition le milieu réactionnel est chauffé à 75 °C pendant 4 h 30. Après refroidissement le mélange est ensuite versé dans 1 200 ml de méthanol. Le précipité formé est filtré et dissous dans 100 ml d'eau distillée. La solution (pH 3,95) est basifiée avec de la soude N jusqu'à pH 8,9. On évapore et le résidu est repris avec 50 ml d'eau distillée. Le pH est contrôlé (7,6) puis la solution est versée dans 1 300 ml de méthanol. Le précipité formé est filtré, repris avec 100 ml d'eau puis la solution ainsi obtenue est traitée avec 30 g de noir animal à 75 °C pendant 1 heure.

On filtre et répète l'opération avec 30 g de noir animal. La solution finale est filtrée sur Millipore 0,5

micron, puis lyophilisée. On obtient 18,2 g d'une poudre de couleur marron.

Caractéristiques analytiques

$\overline{Mn}$ = 7 690 $\overline{Mw}$ = 10 890 D = 1,42 Ma = 9 470
Degré de sulfatation = 1,72
Pourcentage d'acides uroniques = 4,98 % en poids (exprimé en acide glucuronique)
Pourcentage de pentose = 30,4 % en poids (exprimé en xylose monomère)

## Exemple 3

Fractionnement du xylane par solubilisation puis sulfatation

Fractionnement du xylane

30 g de xylane (exemple 1) sont mis en suspension dans 240 ml d'eau distillée.

Le milieu est porté à pH 12 par addition de 6 ml de soude 6 N et abandonné une nuit sous agitation et atmosphère d'azote.

La fraction de xylane insoluble est recueillie par centrifugation.

Les culots sont repris avec de l'eau et les suspensions obtenues sont lyophilisées.

Poids 7,3 g

Pourcentage en acides uroniques : 12,6 % (exprimé en acide glucuronique)

Réaction de sulfatation du xylane insoluble à pH 12

On dissout, sous atmosphère d'azote, 7 g de xylane ci-dessus dans un mélange de 25 ml de pyridine anhydre et 25 ml de DMF anhydre.

La solution refroidie à 0 °C est additionnée goutte à goutte de 16,4 g d'acide chlorosulfonique. Après la fin de l'addition, le mélange réactionnel est chauffé à 75 °C pendant 4 h 30.

Après refroidissement, la solution est versée dans 850 ml de méthanol. Le sel de pyridinium du sulfate de xylane est filtré, rincé avec de l'acétone, et dissous dans 80 ml d'eau (pH 2,85). La solution est basifiée jusqu'à pH 8,1 avec de la soude N, puis évaporée sous vide. Le résidu est repris avec 30 ml d'eau (pH 4,5). On ramène le pH à 11 avec de la soude N et verse la solution dans 1 200 ml de méthanol.

Le précipité crème est filtré, lavé avec de l'éther puis séché sous vide.

Poids obtenu 5 g.

Caractéristiques analytiques

$\overline{Mn}$ = 7 550 $\overline{Mw}$ = 10 270 D = 1,33 Ma = 9 120
Degré de sulfatation = 1,59
Pourcentage d'acides uroniques = 6,10 % en poids (exprimé en acide glucuronique)
Pourcentage de pentoses = 31,3 % en poids (exprimé en xylose monomère).

## Exemple 4

Fractionnement par chromatographie du xylane soluble dans l'eau obtenu à l'exemple 2 puis sulfatation.

Fractionnement du xylane

Le produit de départ utilisé dans ce fractionnement est constitué par la fraction de xylane soluble dans l'eau comme décrit dans l'exemple 2, que l'on obtient par congélation et lyophilisation du filtrat.

On chromatographie 4 g de xylane soluble à 10 % sur 2 colonnes (longueur : 75 cm, diamètre 6,0 cm) garnies de silice poreuse Sphérosil XDA 400 (granulométrie 40-100 mesh, porosité 80 Å), adaptées à un appareil de chromatographie préparative WATERS Prep 500 en utilisant comme éluant de l'acide acétique à 2 % dans de l'eau distillée ; le détecteur est un réfractomètre différentiel.

La courbe réponse obtenue par réfractométrie est découpée en 6 parties et les fractions liquides correspondantes sont lyophilisées. On recommence l'opération cinq fois et on rassemble les fractions homologues.

Bilan cumulé des 6 opérations

Fraction 1 - 1,15 g
Fraction 2 - 3,47 g
Fraction 3 - 3,55 g
Fraction 4 - 7,60 g

Fraction 5 - 5,70 g
Fraction 6 - 0,30 g

Réaction de sulfatation de la fraction 2

On suspend, sous atmosphère d'azote sec, 3,40 g de xylane (fraction 2 ci-dessus), dans 30 ml de diméthylformamide anhydre.

On ajoute 14 g (103 mmoles) de complexe trioxyde de soufre-triméthylamine et l'on chauffe le milieu réactionnel pendant 6 heures à 75 °C. Après refroidissement, le milieu réactionnel est évaporé à sec sous vide. Le résidu obtenu est repris avec 52 ml de soude 2 N et la solution obtenue est à nouveau évaporée à sec. Le résidu est dissous dans 70 ml d'eau distillée, et le pH (initialement à 5,6) est ramené à 8,0 par addition de 15 ml de soude 2 N après évaporation à sec, le résidu est repris avec 70 ml d'eau et la solution obtenue (pH7) est filtré sur millipore 5 µ, puis chromatographiée sur 2 colonnes de silice poreuse (éluant : eau distillée) afin d'éliminer les sels minéraux.

La fraction d'éluant correspondant au sulfate de xylane est lyophilisée.

On obtient 5,30 g de poudre crème.

Caractéristiques analytiques

$\overline{Mn}$ = 9 260 $\overline{Mw}$ = 10 380 D = 1,12 Ma = 9 790
Degré de sulfatation = 1,78
Pourcentage d'acides uroniques = 8,25 % en poids (exprimé en acide glucuronique).
Pourcentage de pentoses = 32 % en poids (exprimé en xylose monomère).

## Exemple 5

Fractionnement par ultrafiltration du xylane soluble dans l'eau obtenu à l'exemple 2, puis sulfatation.

Fractionnement du xylane

On dissout 25 g de la fraction soluble de xylane dans l'eau, comme décrit à l'exemple 2, dans 1 000 ml d'eau distillée. Cette solution est traitée dans un système d'ultrafiltration équipée d'une cartouche de fibres creuses H 1P10-8 de marque AMICON® (seuil de coupure 10.000 daltons) à une pression inférieure ou égale à 1 Bar.

L'opération est terminée lorsque 5 000 ml d'eau distillée ont été consommés par le système.

Le rétentat d'ultrafiltration est alors soutiré du système puis lyophilisé.

On recueille après séchage 4,4 g de poudre crème (rendement : 17,6 % par rapport à la quantité initiale).

Réaction de sulfatation

On suspend, sous atmosphère d'azote sec, 3 g du xylane précédemment isolé, dans 30 ml de diméthylformamide anhydre. On ajoute à la suspension, en plusieurs opérations, 7,90 g (56,7 mmoles) de complexe trioxyde de soufre-triméthylamine. Puis le mélange réactionnel est chauffé à 75 °C pendant 5 h 30.

Après refroidissement, on ajoute 80 ml de soude N puis on évapore à sec sous vide de la trompe à eau.

Le résidu est repris avec 30 ml d'eau distillée, on remonte le pH de la solution obtenue de 5 à 12 par addition de 20 ml de soude N, puis on concentre sous vide. Le résidu d'évaporation est repris par 30 ml d'eau distillée et le pH de la solution obtenue est 9. On ajoute alors de l'acide chlorhydrique N pour ramener le pH à 7, puis la solution est dessalée par élution sur une colonne (L = 90 cm Ø = 4,4 cm) de Sephadex G 15 (détection réfractométrique).

La fraction d'éluat contenant les polysulfates de xylane est recueillie puis lyophilisée.

On récupère après séchage sous vide poussé, à température ambiante, 4,3 g de poudre blanche.

Caractéristiques analytiques

$\overline{Mn}$ = 9 035, $\overline{Mw}$ = 10 550, D = 1,17, Ma = 9 840, degré de sulfatation : 1,64

## Exemple 6

Fractionnement par ultrafiltration du xylane obtenu à l'exemple 2 puis sulfatation.

Fractionnement du xylane

Une solution de 15 g de la fraction du xylane soluble dans l'eau obtenue selon l'exemple 2, dans 1 500 ml d'eau distillée est ultra-filtrée sur un système équipé d'une cartouche de fibres creuses de seuil de coupure 5 000 (type H 1P5-20 marque AMICON®), sous une pression ≤ 1 Bar avec apport continu d'eau distillée.

L'opération est terminée lorsque 8 000 ml d'eau distillée ont été utilisés.

Après concentration de la solution retenue par les fibres creuses elle est soutirée du système, puis lyophilisée.

On récupère 4,5 g (30 % de la quantité initiale) de poudre de couleur crème.

Réaction de sulfatation

On suspend, sous atmosphère d'azote sec, 4 g du xylane obtenu ci-dessus dans 60 ml de diméthylformamide anhydre. On additionne en plusieurs opérations 10,5 g (7,5 mmoles) de complexe trioxyde de soufre-triméthylamine, et on chauffe à 80 °C pendant 6 heures. Après retour à température ambiante, le milieu réactionnel est additionné de 80 ml de soude N, puis évaporé à sec sous vide.

Le résidu est repris par 50 ml d'eau distillée. Le pH de la solution obtenue est porté de 6,25 à 12,4 par addition de 5 ml de soude N. La solution est évaporée à sec, sous vide, puis le résidu est repris par 50 ml d'eau distillée. Le pH est ramené de 10,7 à 7,35 par addition de 1,35 ml d'acide chlorhydrique N. La solution est alors ultra-filtrée sur un système équipé de fibres creuses de seuil de coupure 5 000 (type H 1P5-20, marque AMICON) afin d'éliminer les sels minéraux résiduels, sous une pression inférieure ou égale à 1 Bar.

La solution retenue par le système est lyophilisée. On récupère 6,7 g de poudre blanche.

Caractéristiques analytiques

$\overline{Mn}$ = 8 340, $\overline{Mw}$ = 11 310, D = 1,36, Ma = 9 260
Degré de sulfatation = 1,55
Pourcentage d'acides uroniques : 8,12 % en poids (exprimé en acide glucuronique)
Pourcentage de Pentoses : 38,3 % en poids (exprimé en xylose monomère)

Exemple 7

Fractionnement par précipitation de la fraction de xylane soluble dans l'eau obtenue à l'exemple 2 puis sulfatation.

Fractionnement du xylane

On dissout sous agitation magnétique, 40 g de la fraction de xylane soluble dans l'eau obtenue selon l'exemple 2 dans 400 ml d'eau distillée.

On ajoute à cette solution, en maintenant l'agitation, 200 ml d'éthanol à 95°.

Après une demi-heure d'agitation, la suspension obtenue est centrifugée. Les culots de centrifugation sont décantés, repris par de l'eau et récupérés par lyophilisation. On obtient 2,4 g de produit de couleur crème.

Réaction de sulfatation

On suspend sous agitation magnétique et atmosphère d'azote sec, 2 g de la fraction de xylane obtenu ci-dessus dans 25 ml de diméthylformamide anhydre.

On ajoute à ce mélange 5,25 g (0,037 moles) de complexe trioxyde de soufre-triméthylamine, et l'on chauffe à 75 °C pendant 5 h 30. Après retour à température ambiante, on ajoute au milieu réactionnel 52 ml de soude N, et l'on évapore à sec sous vide. Le résidu ainsi obtenu est repris par 40 ml d'eau distillée, et le pH de la solution obtenue est porté de 6 à 13 par addition de 10 ml de soude N. On évapore à sec sous vide et le résidu est repris par 40 ml d'eau distillée. Cette solution est ramenée à pH 7 avec de l'acide chlorhydrique N puis filtrée sur Millipore 8 μ, et enfin éluée sur une colonne de Sephadex G15 pour éliminer les résidus minéraux.

La fraction d'éluat correspondant au polysulfate de xylane est recueillie et lyophilisée.

On obtient 3,75 g de poudre blanche.

Caractéristiques analytiques

$\overline{Mn}$ = 10 040, $\overline{Mw}$ = 11 800, D = 1,18, Ma = 10 685
Degré de sulfatation = 1,72

Exemple 8

Fractionnement par solubilisation du xylane obtenu à l'exemple 1 puis sulfatation

Fractionnement du xylane

On ajoute, sous agitation magnétique, 150 g de xylane obtenu selon le procédé décrit à l'exemple 1 dans 1 000 ml d'eau distillée thermostatée à 50 °C. Après 8 heures d'agitation, on laisse revenir à température ambiante, et la fraction insoluble est récupérée par centrifugation.

Les culots de centrifugation repris par un peu d'eau distillée sont rassemblés et lyophilisés.

On récupère 38,3 g de poudre de couleur crème.

Réaction de sulfatation

On met en suspension, sous agitation magnétique et sous atmosphère d'azote sec, 5 g de xylane obtenu ci-dessus, dans 50 ml de diméthylformamide anhydre. La suspension est additionnée de 13,5 g (94,5 mmoles) de complexe trioxyde de soufre-triméthylamine et l'on chauffe le milieu réactionnel à 75 °C pendant 8 h. Après retour à température ambiante le mélange est basifié avec 150 ml de soude N jusqu'à pH 8 puis évaporé à sec sous vide. Le résidu est repris avec 50 ml d'eau distillée et le pH de la solution est ramené de 8,3 à 6,85 par addition de 0,9 ml d'acide chlorhydrique N.

La solution est alors ultrafiltrée sur un système à fibres creuses équipé d'une cartouche à seuil de coupure 5 000 Daltons, de (type H 1-P5-20, marque AMICON), sous une pression inférieure ou égale à 1 Bar, afin d'éliminer les sels minéraux.

Le rétentat est ensuite lyophilisé.

On recueille 7,9 g de poudre crème.

Caractéristiques analytiques

$\overline{Mn}$ = 6 670, $\overline{Mw}$ = 11 430, D = 1,72, Ma = 9 390

Degré de sulfatation : 1,79

Pourcentage d'acides uroniques : 5,1 % en poids (exprimé en acide glucuronique)

Pourcentage de Pentoses : 39,2 % en poids (exprimé en xylose monomère)

Exemple 9

Fractionnement du polysulfate de xylane (SP 54) par chromatographie.

Une colonne de chromatographie (L = 60 cm, Ø = 4,4 cm) est remplie de cellulose échangeuse d'ions (WHATMAN DE 52) préalablement conditionnée avec un mélange tampon (AcONa 0,2 M, AcOH 0,06 M) à pH 6,5. Le débit de l'éluant est contrôlé par une pompe péristaltique et l'analyse des éluats est réalisé par un détecteur U. V. (λ = 254 nm). On injecte sur la colonne 20 g de polysulfate de xylane (SP 54), dissous dans 60 ml de tampon initial, puis on élue avec le même tampon jusqu'à stabilisation du produit sur le gel échangeur.

On élue ensuite avec une succession de solutions de chlorure de sodium, de concentrations croissantes.

Les fractions d'éluat correspondant à chaque solution éluante sont recueillies et lyophilisées.

Succession d'éluants utilisés :

0,3 M NaC1 - F1
0,4 M NaC1 - F2
0,5 M NaC1 - F3
0,6 M NaC1 - F4
0,7 M NaC1 - F5
0,8 M NaC1 - F6
0,9 M NaC1 - F7
1,0 M NaC1 - F8
1,2 M NaC1 - F9

Chaque fraction est dessalée, par chromatographie sur colonne de silice poreuse (éluant : eau distillée) et la fraction correspondant aux sulfates de polysaccharides purifiés est récupérée par lyophilisation. L'opération est recommencée plusieurs fois pour pouvoir obtenir des quantités suffisantes de chaque produit, par rassemblement des fractions homologues.

Bilan global de quatre opérations :

F1 4,2 g (tampon 0,3 M NaC1)

F2 5,3 g (tampon 0,4 M NaC1)
F3 4,65 g (tampon 0,5 M NaC1)
F4 5,25 g (tampon 0,6 M NaC1)
F5 6,45 g (tampon 0,7 M NaC1)
F6 6,30 g (tampon 0,8 M NaC1)
F7 6,65 g (tampon 0,9 M NaC1)
F8 3,3 g (tampon 1,0 M NaC1)
F9 0,65 g (tampon 1,2 M NaC1)

Caractéristiques analytiques des fractions sélectionnées :

| | $\overline{Mn}$ | $\overline{Mw}$ | Disp. | Ma | D.S. | % acides uroniques | % Pentoses |
|---|---|---|---|---|---|---|---|
| F6 | 8120 | 9090 | 1,2 | 8790 | 1,58 | 5,38 | 38,04 |
| F7 | 10500 | 12050 | 1,14 | 10960 | 1,71 | 4,70 | 38,66 |
| F8 | 11320 | 12660 | 1,12 | 11500 | 1,69 | 4,68 | 38,04 |
| F9 | 11740 | 13240 | 1,12 | 11940 | 1,71 | 2,06 | 35,49 |

Exemple 10

Fractionnement du polysulfate de xylane (SP 54) par ultrafiltration.

Une solution de 30 g de polysulfate de xylane (SP 54) dans 600 ml d'eau distillée est ultrafiltrée dans un système équipé d'une membrane AMICON SYMS (seuil de coupure 5 000) et d'une réserve d'eau distillée. La pression de travail est assurée par de l'azote sous 4 Bars. Après avoir recueilli 800 ml d'ultrafiltrat, le contenu de la cellule est récupéré et lyophilisé.

On obtient 11,75 g de poudre blanche (soit 39,1 % du poids initial).

Caractéristiques analytiques :

$\overline{Mn}$ = 7 180, $\overline{MW}$ = 9 500, D = 1,32 ; Ma = 7 890
Degré de sulfatation : 1,71

Exemple 11

Fractionnement du polysulfate de xylane (SP 54) par chromatographie.

On injecte 3 g de polysulfate de xylane (SP 54) dans 10 ml d'eau distillée sur un système chromatographique composé de deux colonnes en série (L = 75 cm, Ø = 6 cm) garnies de silice poreuse Spherosil XOA 400 (granulométrie 40-100 mesh, porosité 80 Å) avec un débit d'éluant (eau distillée) de 40 ml/mn.

La détection est assurée par un réfractomètre différentiel.

La partie d'éluant contenant le polysulfate de xylane est fractionnée en quatre volumes.

L'opération est recommencée 15 fois et les fractions homologues sont regroupées et lyophilisées.

Bilan des 16 opérations :

F1 5,9 g (rendement en poids 12,3 %)
F2 5,7 g (rendement en poids 12 %)
F3 13,6 g (rendement en poids 28,3 %)
F4 15,74 g (rendement en poids 32,8 %)

Caractéristiques analytiques de la fraction de plus haut poids moléculaire (fraction 1)

$\overline{Mn}$ = 9 370, $\overline{Mw}$ = 11 180, D = 1,19, Ma = 9 840
Degré de sulfatation = 1,73

Exemple 12

Fractionnement du polysulfate de xylane (SP 54) par ultrafiltration.

On soumet une solution de 20 g de polysulfate de xylane (SP 54) dans 300 ml d'eau distillée à une ultrafiltration sur un système équipé de fibres creuses de seuil de coupure 5 000 Daltons (type H 1 P5-20 marque AMICON), sous une pression inférieure ou égale à 1 Bar.

Après que le système ait absorbé 1 500 ml d'eau distillée, la solution des hauts poids moléculaires retenus par les fibres creuses est soutirée du système et lyophilisée.

On recueille 6,7 g de poudre blanche (rendement : 33,5 % de la quantité initiale).

Caractéristiques analytiques

$\overline{MN}$ = 7 160, $\overline{Mw}$ = 8 820, D = 1,23, Ma = 7 390
Degré de sulfatation = 1,71

Exemple 13

Fractionnement du polysulfate de xylane (SP 54) par ultrafiltration.

On soumet une solution de 60 g de polysulfate de xylane (SP 54) dans 1 000 ml d'eau distillée à une ultrafiltration sur un système équipé de fibres creuses de seuil de coupure 10 000 (type H 1 P10-8, marque AMICON), sous une pression inférieure ou égale à 1 Bar.

Après que le système ait absorbé 5 000 ml d'eau distillée, la solution des hauts poids moléculaires retenue par les fibres creuses est soutirée du système et lyophilisée.

On recueille 3,6 g de poudre blanche (rendement = 6 % en poids de la quantité initiale).

Caractéristiques analytiques :

$\overline{Mn}$ = 9 500, $\overline{Mw}$ = 11 720, D = 1,20
Degré de sulfatation = 1,63

Exemple 14

Fractionnement du polysulfate de xylane (SP 54) par ultrafiltration.

Une solution de 30 g de polysulfate de xylane (SP 54) dissous dans 500 ml de $NaNO_3$ 1M est ultrafiltrée sur le système déjà utilisé dans l'exemple 10, équipé d'une membrane identique de seuil de coupure 5 000 (type 5YM5 marque AMICON), mais la réserve d'éluant est remplie avec une solution de nitrate de sodium 1M.

La pression de travail est assurée par de l'azote sous 4 Bars. Après avoir recueilli 1 600 ml d'ultrafiltrat, le contenu de la cellule est récupéré, puis injecté en plusieurs opérations, sur une colonne préparative (L = 45 cm, Ø =10 cm), garnie de Sephadex G25, éluée à l'eau distillée, afin d'éliminer le nitrate de sodium.

Les fractions éluant contenant les polymères purifiés sont rassemblées et lyophilisées.

On recueille 6,87 g de poudre blanche (rendement : 22,9 % en poids de la quantité initiale).

Caractéristiques analytiques

$\overline{Mn}$ = 9 850, $\overline{Mw}$ = 11 815, D = 1,20
Degré de sulfatation = 1,70

Exemple 15

Fractionnement du polysulfate de xylane (SP 54) par ultrafiltration.

Une solution de 1,2 kg de polysulfate de xylane (SP 54) dans 18 litres d'eau déminéralisée (concentration en poids 6,66 %) est ultrafiltrée dans un système équipé d'une cartouche de fibres creuses ROMICON 22-20 PM-5 (Longueur 63 cm, surface développée des filtres 2,4 $cm^3$) et d'une admission d'eau déminéralisée permettant de garder constant, pendant l'opération, le volume de la solution à traiter.

La pompe de recirculation de la solution est réglée pour assurer une pression moyenne de 1,45 Bar et un débit moyen de perméat de 150 ml par minute. Lorsque 42 litres d'ultrafiltrat (2,5 volumes par rapport au volume de solution initiale) ont été obtenus, on soutire le rétentat du système et rince le corps de pompe et l'intérieur des fibres creuses avec 3 litres d'eau déminéralisée.

La solution (21 litres) constituée par le retentat et la solution de rinçage est alors retraitée par le système équipé cette fois d'une cartouche de fibres creuses ROMICON 30-20 PM-10 (longueur 63 cm surface développée des fibres 2,8 $cm^3$).

Dans un premier temps la solution est concentrée de moitié (Recueil de 10,5 litres d'ultrafiltrat). Puis l'admission d'eau est ouverte et l'on ultrafiltre à volume de rétentat constant sous 1,4 Bar de pression jusqu'à obtention d'un volume total d'ultrafiltrat de 52 litres (ultrafiltrat de concentration compris). Ce perméat final de 52 litres est alors concentré jusqu'à 22 litres sur le même système équipé d'une cartouche ROMICON 15-43 PM2 (Longueur 63 cm, surface développée des fibres 1,4 $cm^3$) puis lyophilisée.

On recueille 315 g de poudre blanche (Rendement 26,2 % en poids de la quantité initiale).

Caractéristiques analytiques

$\overline{Mn}$ = 6 700 $\overline{Mw}$ = 7 850 D = 1,17 Ma = 7 500

Degré de sulfatation = 1,76

Les résultats des essais pharmacologiques, qui sont rapportés ci-après, ont permis de mettre en évidence les intéressantes propriétés des produits de l'invention, c'est-à-dire les différentes fractions qui ont été obtenues à partir du SP 54.

L'invention a donc encore pour objet un médicament ayant en particulier des propriétés antithrombotique et hypolipémiante caractérisé en ce qu'il contient, à titre de principe actif, au moins une des fractions préparées par le procédé de l'invention et répondant à la formule (I).

L'étude pharmacologique a porté sur les actions anticoagulante, anti-thrombotique, fibrinolytique, lipolytique et le temps de saignement ; ces études ont été effectuées comparativement, d'une part à l'héparine, et d'autre part au SP 54.

— L'action anticoagulante est déterminée par le temps de céphaline activé (CAEN J., LARRIEU M.J., SAMAMA M. In : L'hémostase : méthodes d'exploration et diagnostic pratique. Ed. : l'Expansion Scientifique Française, 1975, p. 169).

Le temps de céphaline activé (TCA), mesure la recalcification du plasma déplaquetté en présence d'un optimum de lipides (céphaline) et de célite qui active de manière standardisée les facteurs de la phase de contact (les facteurs XII, XI, IX). Il mesure donc la formation de prothrombinase endogène à l'exception des facteurs plaquettaires remplacés par la céphaline.

— L'action antithrombotique est appréciée par l'activité anti Xa (YIN E.T., WESSLER S., BUTLER J.V. — J. Lab. Clin. Med., 1973, *81*, 298-310). Le temps de yin mesure l'action neutralisante du SP 54 et de ses fractions vis à vis du facteur Xa en présence d'un excès d'antithrombine III et facteur Xa.

L'activité antithrombotique est aussi testée à l'aide d'un modèle de thrombose expérimentale sur fil de soie introduit dans un shunt artérioveineux chez le rat (UMETSU T., SANAI K. — Thromb. Haemost, 1978, *39*, 74-83).

— L'activité fibrinolytique est mesurée par le temps de lyse des euglobulines plasmatiques (KLUFT C. — Haemostasis, 1976, *5*, 136).

Le temps de lyse des euglobulines plasmatiques est une méthode sensibilisée permettant d'apprécier l'action fibrinolytique globale du SP 54 et de ses fractions en l'absence des inhibiteurs physiologiques de la fibrinolyse, éliminés par dilution et acidification du plasma.

— L'action lipolytique est mesurée par l'activité lipoprotéine lipase circulante (NIKKILA E.A., HUTTUNEN J.K., EHNHOLM C. — Métabolism, 1977, *26*, 179).

La détermination de l'activité lipoprotéine lipase circulante est une mesure indirecte de l'impact du SP 54 et de ses fractions sur le catabolisme des triglycérides (pouvoir clarifiant). L'activité enzymatique est déterminée par sa capacité d'hydrolyser le substrat 14C-trioleine en 14C-acide oléique.

— Le temps de saignement est déterminé après section transversale de la queue du rat (STELLA L., DONATI M.B., DE GAETANO G. — Thromb. Res., 1975, *7*, 709-716).

Pour ces différentes expérimentations, des rats mâles (Sprague Dawley) d'un poids de 200 g à 300 g ont été utilisés.

Ces animaux sont anesthésiés au pentobarbital (50 mg/kg) par la voie intrapéritonéale et les produits à tester sont administrés par la voie intraveineuse (veine du pénis). Quinze minutes après l'administration des produits, les échantillons sanguins sont prélevés à l'aorte abdominale et rendus incoagulables par une solution de citrate trisodique à 3,8 p. 100 (1 volume d'anticoagulant pour 9 volumes de sang) ou de l'héparine (25 U/ml de sang).

Immédiatement après le prélèvement sanguin, les échantillons sont centrifugés à 2 000 × g pendant 15 minutes à 4 °C, pour la préparation du plasma citraté qui est utilisé pour les différents dosages.

(Voir Tableau I page 12)

Les résultats sont rassemblés dans les tableaux 1 et 2 ; leur analyse statistique a été effectuée :
a) selon le test de STUDENT (groupe de comparaison : SP 54)
b) selon le test U de MANN et WHITNEY (groupe de comparaison : témoins).

NS : non significatif
X : p 0,05
XX : p 0,01
XXX : p 0,001

Tableau I

| PRODUITS | Ma | COAGULATION SANGUINE (a) | | |
|---|---|---|---|---|
| | | T.C.A. | | ANTI-Xa |
| | | 7,5 mg/kg/IV % prolongation | 5 mg/kg/IV % prolongation | 5 mg/kg/IV % prolongation |
| SP 54 | 6 000 | 104 | 200 | 25 |
| HEPARINE | 20 000 | 1 178 | incoagulable | 506 |
| Exemple 2 | 9 470 | 129 X | 298 X | 57 XX |
| Exemple 3 | 9 120 | 155 XX | 512 XXX | 183 XXX |
| Exemple 4 | 9 790 | 178 XXX | [illegible] XXX | 91 XXX |
| Exemple 5 | 9 840 | 214 XXX | 1 090 XXX | 79 XX |
| Exemple 6 | 9 260 | 166 XX | 1 100 XXX | 112 XXX |
| Exemple 7 | 10 685 | 190 XXX | 591 XXX | 70 XXX |
| Exemple 8 | 9 380 | 134 X | 461 XXX | 76 XX |
| Exemple 9 F6 | 8 790 | 102 (NS) | 183 (NS) | 67 XX |
| Exemple 9 F7 | 10 950 | 185 XXX | 442 XXX | 74 XX |
| Exemple 9 F8 | 11 500 | 212 XXX | 631 XXX | 104 XXX |
| Exemple 9 F9 | 11 940 | 243 XXX | 723 XXX | 112 XXX |
| Exemple 10 | 7 890 | 156 XXX | 486 XXX | 80 XXX |
| Exemple 11 | 9 840 | 248 XXX | 844 XXX | 66 XX |
| Exemple 12 | 7 390 | 149 (NS) | 369 XX | 67 XX |
| Exemple 13 | 10 780 | 281 XXX | 1 100 XXX | 126 XXX |
| Exemple 14 | 10 190 | 173 XX | 634 XXX | 64 XX |
| Exemple 15 | 7 500 | 171 X | 352 (NS) | 41 X |

Tableau 2

| PRODUITS | Ma | FIBRINOLYSE (a) 2,5mg/kg/IV % d'activation | LIPOPROTEINE LIPASE (a) 2,5mg/kg/IV % de libération | THROMBOSE FIL DE SOIE (b) 5 mg/kg/IV % d'inhibition | TEMPS DE SAIGNEMENT (b) 5 mg/KG/IV Facteur d'allongement |
|---|---|---|---|---|---|
| SP 54 | 6 000 | 48 | 59 | - 53 XX | X 1,6 * |
| HEPARINE | 20 000 | 40 | 64 | - 57 XX | > X 10 XX |
| Exemple 2 | 9 470 | 57 XX | 84 XX | - 56 XX | X 1,6 * |
| Exemple 3 | 9 120 | 57 XX | 105 XXX | - 85 XX | X 4,2 XX |
| Exemple 4 | 9 790 | 65 XXX | 101 XXX | - 96 XX | |
| Exemple 5 | 9 840 | 61 XXX | 86 XX | - 86 XX | |
| Exemple 6 | 9 260 | 50 (NS) | 97 XXX | - 26 (NS) | X 3,0 XX |
| Exemple 7 | 10 685 | 37 XX | 84 XX | | |
| Exemple 8 | 9 380 | 46 (NS) | 77 XX | -100 XX | > X 10 XX |
| Exemple 9 F6 | 8 790 | 36 XX | 64 (NS) | | |
| Exemple 9 F7 | 10 960 | 41 XX | 77 XXX | | |
| Exemple 9 F8 | 11 500 | 60 XXX | 92 XXX | - 42 XX | X 1,4 (NS) |
| Exemple 9 F9 | 11 940 | 62 XXX | 115 XXX | | |
| Exemple 10 | 7 890 | 58 XX | 150 XXX | - 85 XX | X 2,1 * |
| Exemple 11 | 9 840 | 63 XXX | 78 XX | - 79 XX | X 2,7 X |
| Exemple 12 | 7 390 | 42 (NS) | 96 XXX | | |
| Exemple 13 | 10 780 | 48 (NS) | 94 XXX | | |
| Exemple 14 | 10 190 | 35 XX | 65 XX | | |
| Exemple 15 | 7 500 | 41 (NS) | 38 X | - 54 XXX | X 2,2 X |

Les travaux qui viennent d'être rapportés ont mis en évidence les intéressantes propriétés anti-thrombotique et hypolipémiante des produits de l'invention.

En effet, bien que produisant une action anti-thrombotique importante, l'effet anticoagulant du médicament de l'invention est faible, réduisant ainsi notamment les risques d'hémorragie qui accompagnent ce type de médicaments.

Le médicament de l'invention peut être présenté sous des formes administrables par les voies orale, parentérale, rectale et locale.

Chaque dose unitaire contient avantageusement de 0,010 g à 0,250 g, les doses administrables journellement pouvant varier de 0,010 g à 0,500 g en fonction de l'âge et de la gravité de l'affection traitée.

On donnera ci-après à titre d'exemple non limitatif quelques formulations pharmaceutiques du médicament de l'invention.

1. Comprimés

| | |
|---|---|
| Fraction n° 2 | 0,050 g |
| Excipient q.s.p. | 1 comprimé |

2. Gélules

| | |
|---|---|
| Fraction n° 6 | 0,075 g |
| Excipient q.s.p. | 1 gélule |

3. Ampoules injectables

| | |
|---|---|
| Fraction n° 8 | 0,100 g |
| Solvant isotonique q.s.p. | 1 ampoule de 2 ml |

4. Pommade

| | |
|---|---|
| Fraction n° 10 | 0,150 g |
| Excipient q.s.p. | 1 tube de 30 g |

Par leur action sur les différents stades de la coagulation sanguine et de la fibrinolyse, ces médicaments peuvent être utilisés avec profit, en cures prolongées et répétées dans le traitement préventif ou curatif d'accidents thrombo-embolique veineux ou artériels dans les cas de phlébites, embolies pulmonaires et autres situations cliniques du même type, angines de poitrine, infarctus du myocarde, artérites chroniques des membres inférieurs, troubles circulatoires superficiels et ulcères des jambes.

Ils peuvent également être utilisés pour prévenir les thromboses dans les circuits extra-corporels (hémodialyses rénales).

En outre, leur action sur la lipoprotéine-lipase (facteur clarifiant) les rend particulièrement utiles dans le traitement des dyslipémies athérogènes.

Enfin, ces médicaments peuvent également être utilisés avec profit dans le traitement de certaines maladies inflammatoires, telles que polyarthrite rhumatoïde, arthroses et ostéoarthrites.

**Revendications** (pour les Etats contractants : BE, CH, DE, FR, GB, IT, LI, LU, NL, SE)

1. Fractions de sulfates de xylanes de formule suivante

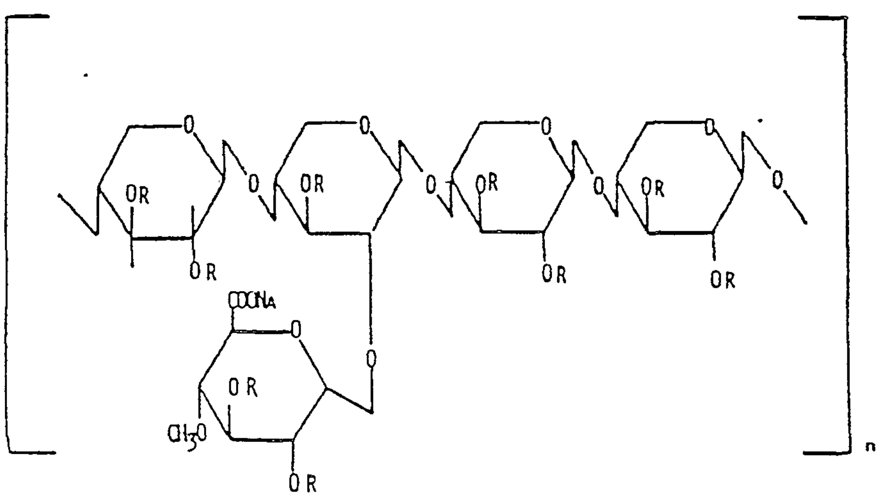

(I)

caractérisée par :

— une masse molaire apparente comprise entre 7 000 et 12 000,

— un degré de sulfatation compris entre 1,5 et 2,0,

— un pourcentage en acide uronique compris entre 0 et 10,

— et un pourcentage en pentose compris entre 30 et 40.

2. Procédé de préparation des composés selon la revendication 1, caractérisé en ce que le polysulfate de xylane SP 54, dont la masse moléculaire apparente est inférieure à 7 000, est soumis à des fractionnements par chromatographie sur colonne ou par ultra-filtration.

3. Procédé de préparation selon la revendication 2, caractérisé en ce que, selon une variante, le fractionnement peut être effectué après désulfatation du SP 54 et les fractions alors obtenues sont ensuite sulfatées de nouveau.

4. Procédé de préparation selon la revendication 3, caractérisé en ce que la désulfatation est effectuée par traitement avec du diméthylformamide additionné de 3 p. 100 à 10 p. 100 d'eau à des températures comprises entre 50 °C et 100 °C.

5. Procédé de préparation selon l'une des revendications 3 ou 4, caractérisé en ce que la sulfatation est effectuée soit par l'acide chlorosulfonique en présence de pyridine et de dimétylformamide, soit par le complexe trioxyde de soufre-triméthylamine dans le dimétylformamide.

6. Médicament ayant notamment des activités antithrombotique et hypolipémiante, caractérisé en ce qu'il contient, à titre de principe actif, un composé tel que défini à la revendication 1.

7. Médicament selon la revendication 6, caractérisé en ce qu'il est présenté sous forme appropriée à l'administration orale, parentérale, rectale ou locale, associé à des véhicules ou excipients thérapeutiquement acceptables.

8. Médicament selon la revendication 6, caractérisé en ce qu'il est présenté sous forme de doses unitaires contenant chacune de 0,010 g à 0,250 g de principe actif.

**Revendications** (pour l'Etat contractant AT)

1. Procédé de préparation de sulfates de xylanes de formule suivante

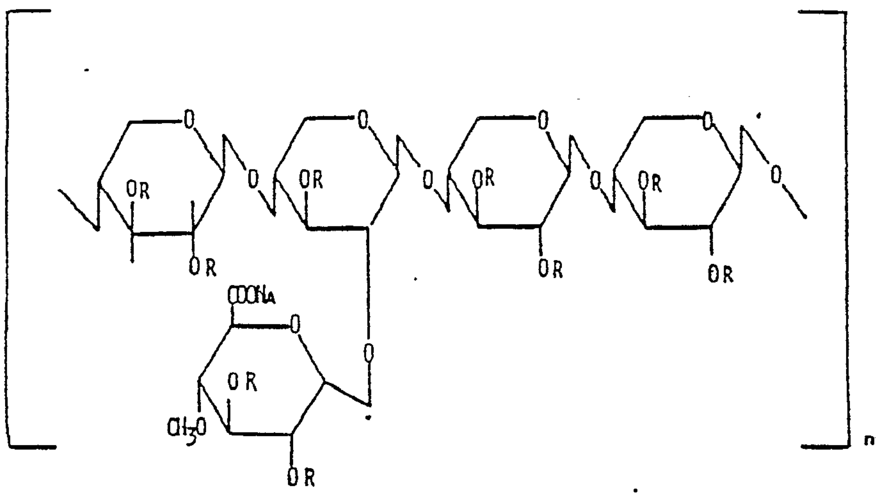

(I)

et ayant :

— une masse molaire apparente comprise entre 7 000 et 12 000,

— un degré de sulfatation compris entre 1,5 et 2,0,

— un pourcentage en acide uronique compris entre 0 et 10,

— et un pourcentage en pentose compris entre 30 et 40, caractérisé en ce que le polysulfate de xylane SP 54, dont la masse moléculaire apparente est inférieure à 7 000, est soumis à des fractionnements par chromatographie sur colonne ou par ultrafiltration.

2. Procédé de préparation selon la revendication 1, caractérisé en ce que, selon une variante, le fractionnement peut être effectué après désulfatation du SP 54 et les fractions alors obtenues sont ensuite sulfatées de nouveau.

3. Procédé de préparation selon la revendication 2, caractérisé en ce que la désulfatation est effectuée par traitement avec du diméthylformamide additionné de 3 p. 100 à 10 p. 100 d'eau à des températures comprises entre 50 °C et 100 °C.

4. Procédé de préparation selon l'une des revendications 2 ou 3, caractérisé en ce que la sulfatation est effectuée soit par l'acide chlorosulfonique en présence de pyridine et de diméthylformamide, soit par le complexe trioxyde de soufre-triméthylamine dans le diméthylformamide.

5. Procédé de préparation d'un médicament ayant notamment des activités antithrombotique et hypolipémiante, caractérisé en ce que l'on utilise un composé tel que défini à la revendication 1.

**Claims** (for the Contracting States : BE, CH, DE, FR, GB, IT, LI, LU, NL, SE)

1. Fractions of xylane sulfates having the following formula

(See Formula page 16)

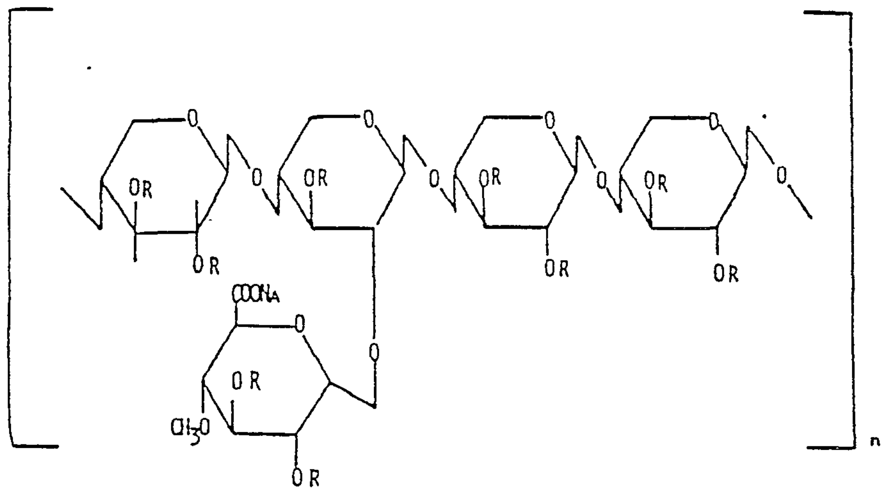

(I)

characterized by :

— an apparent molar weight comprised between 7 000 and 12 000,
— a sulfatation degree comprised between 1.5 and 2.0,
— a percent uronic acids content comprised between 0 and 10,
— and a percent pentoses content comprised between 30 and 40.

2. Process for the preparation of the compounds of claim 1, characterized in that xylane polysulfate SP 54, whose apparent molecular weight is below 7 000, is fractionated by column chromatography or by ultrafiltration.

3. Process as claimed in claim 2, characterized in that, according to a modification, the fractionation may be after desulfatation of SP 54, after which the resulting fractions are again sulfated.

4. Process as claimed in claim 3, characterized in that the desulfatation is effected by treatment with dimethylformamide added with 3 % to 10 % water, at temperatures comprised between 50 °C and 100 °C.

5. Process as claimed in any one of claims 3 or 4 characterized in that the sulfatation is effected either with chlorosulfonic acid in the presence of pyridine and dimethylformamide, or with the complex sulfur trioxide-trimethylamine within dimethylformamide.

6. Medicament having in particular antithrombosis and hypolipaemic activities, characterized in that it contains, as active ingredient, a compound as defined in claim 1.

7. Medicament as claimed in claim 6, characterized in that it is formulated in a form suitable for oral, parenteral, rectal or topical administration, in combination with therapeutically acceptable vehicles or excipients.

8. Medicament as claimed in claim 6, characterized in that it is presented in unit dosage form, each unit dose containing 0.010 g to 0.250 g active ingredient.

**Claims** (for the Contracting State AT)

1. Process for the preparation of xylane sulfates having the following formula

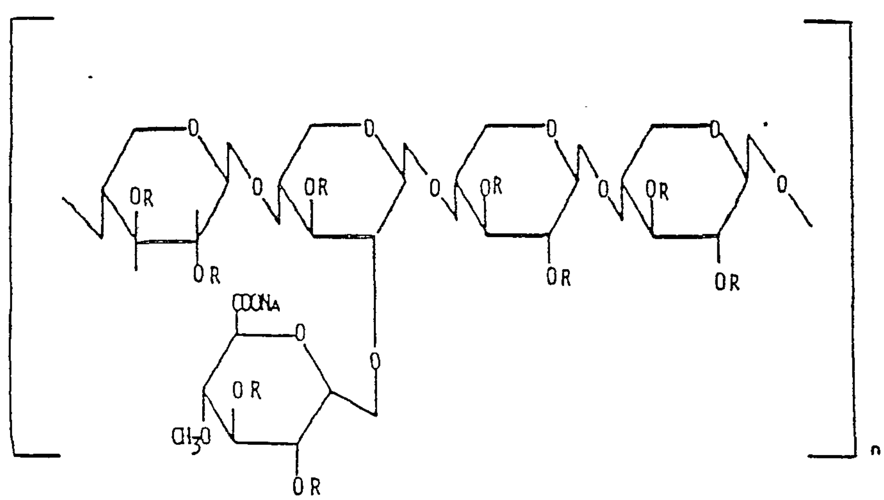

(I)

and having :

— an apparent molar weight comprised between 7 000 and 12 000,
— a sulfatation degree comprised between 1.5 and 2.0,
— a percent uronic acids content comprised between 0 and 10,
— and a percent pentoses content comprised between 30 and 40, characterized in that xylane polysulfate SP 54, whose apparent molecular weight is below 7 000, is submitted to fractionation by column chromatography or by ultrafiltration.

2. Process as claimed in claim 1, characterized in that, according to a modification, the fractionation may be effected after desulfatation of SP 54, after which the resulting fractions are again sulfated.

3. Process as claimed in claim 2, characterized in that the desulfatation is effected by treatment with dimethylformamide added with 3 % to 10 % water, at temperatures comprised between 50 °C and 100 °C.

4. Process as claimed in any one of claims 3 or 4, characterized in that the sulfatation is effected either with chlorosulfonic acid in the presence of pyridine and dimethylformamide, or with the complex sulfur trioxide-trimethylamine within dimethylformamide.

5. Process for the preparation of a medicament having in particular anti-thrombosis and hypolipaemic activities, characterized in that a compound as defined in claim 1 is used.

**Patentansprüche** (für die Vertragsstaaten : BE, CH, DE, FR, GB, IT, LI, LU, NL, SE)

1. Fraktionen von Sulfaten von Xylanen der folgenden Formel

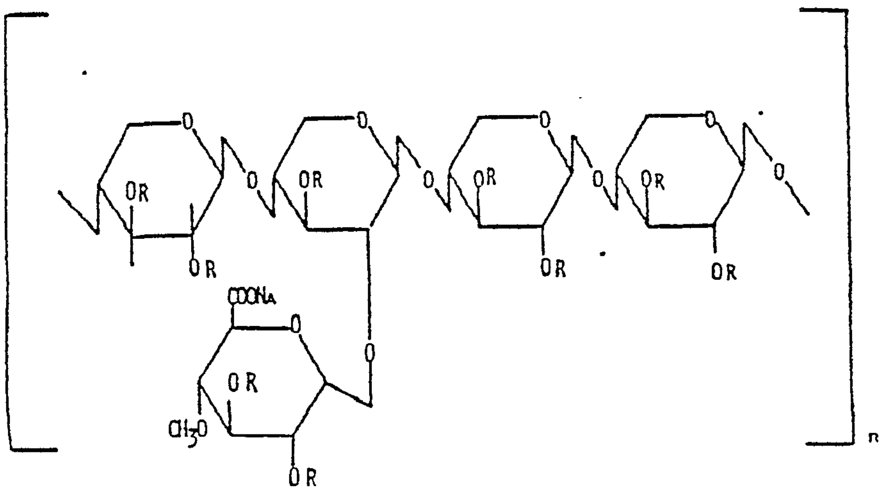

(I)

gekennzeichnet durch :
— ein scheinbares Molekulargewicht zwischen 7 000 und 12 000,
— einen Sulfatierungsgrad zwischen 1,5 und 2,0,
— einen Prozentsatz an Uronsäure zwischen 0 und 10 und
— einen Prozentsatz an Pentose zwischen 30 und 40.

2. Verfahren zur Herstellung von Verbindungen nach Anspruch 1, dadurch gekennzeichnet, daß das Xylanpolysulfat SP 54, dessen scheinbares Molekulargewicht unter 7 000 liegt, Fraktionierung durch Säulenchromatographie oder durch Ultrafiltration unterworfen wird.

3. Verfahren zur Herstellung nach Anspruch 2, dadurch gekennzeichnet, daß gemäß einer Variante die Fraktionierung nach Desulfatierung von SP 54 durchgeführt werden kann und die hierbei erhaltenen Fraktionen anschließend erneut sulfatiert werden.

4. Verfahren zur Herstellung nach Anspruch 3, dadurch gekennzeichnet, daß die Desulfatierung durch Behandlung mit Dimethylformamid, dem 3 % bis 10 % Wasser zugesetzt worden ist, bei Temperaturen zwischen 50 °C und 100 °C durchgeführt wird.

5. Verfahren zur Herstellung nach einem der Ansprüche 3 oder 4, dadurch gekennzeichnet, daß die Sulfatierung entweder mit Chlorsulfonsäure in Gegenwart von Pyridin und von Dimethylformamid oder mit dem Schwefeltrioxid-Trimethylamin-Komplex in Dimethylformamid durchgeführt wird.

6. Medikament mit insbesondere anti-thrombotischen Wirkungen und Hypolipämie-Wirkungen, dadurch gekennzeichnet, daß es als Wirkstoff eine Verbindung, wie sie in Anspruch 1 definiert wurde, enthält.

7. Medikament nach Anspruch 6, dadurch gekennzeichnet, daß es in einer für die orale, parenterale, rektale oder lokale Verabreichung geeigneten Form in Verbindung mit therapeutisch unbedenklichen Trägern oder Hilfsstoffen dargeboten wird.

8. Medikament nach Anspruch 6, dadurch gekennzeichnet, daß es die Form von Einheitsdosen hat, die jeweils 0,010 g bis 0,250 g Wirkstoff enthalten.

**Patentansprüche** (für den Vertragsstaat AT)

1. Verfahren zur Herstellung von Sulfaten von Xylanen der folgenden Formel

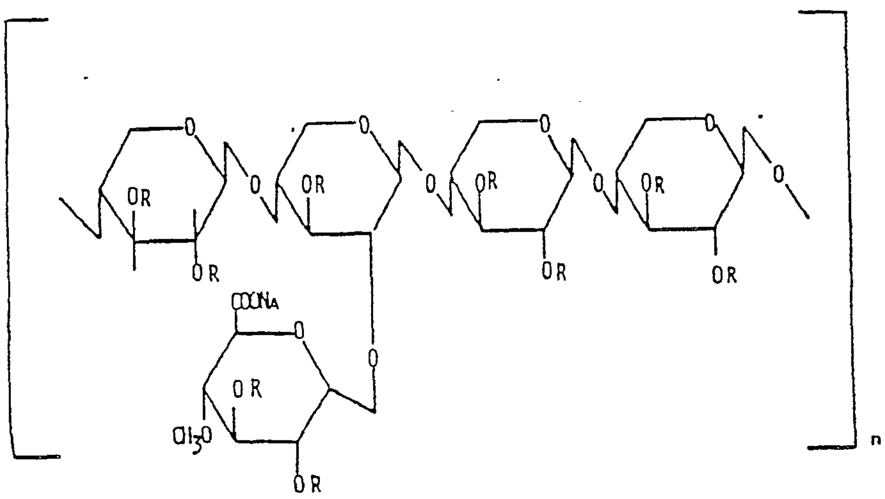

(I)

gekennzeichnet durch :
- ein scheinbares Molekulargewicht zwischen 7 000 und 12 000,
- einen Sulfatierungsgrad zwischen 1,5 und 2,0,
- einen Prozentsatz an Uronsäure zwischen 0 und 10 und
- einen Prozentsatz an Pentose zwischen 30 und 40,

dadurch gekennzeichnet, daß das Xylanpolysulfat SP 54, dessen scheinbares Molekulargewicht unter 7 000 liegt, Fraktionierungen durch Säulenchromatographie oder durch Ultrafiltration unterworfen wird.

2. Herstellungsverfahren nach Anspruch 1, dadurch gekennzeichnet, daß gemäß einer Variante die Fraktionierung nach Desulfatierung von SP 54 durchgeführt werden kann und die hierbei erhaltenen Fraktionen anschließend erneut sulfatiert werden.

3. Herstellungsverfahren nach Anspruch 2, dadurch gekennzeichnet, daß die Desulfatierung durch Behandlung mit Dimethylformamid, dem 3 % bis 10 % Wasser zugesetzt worden sind, bei Temperaturen zwischen 50 °C und 100 °C durchgeführt wird.

4. Herstellungsverfahren nach einem der Ansprüch 2 oder 3, dadurch gekennzeichnet, daß die Sulfatierung entweder mit Chlorsulfonsäure in Gegenwart von Pyridin und Dimethylformamid oder mit dem Schwefeltrioxid-Trimethylamin-Komplex in Dimethylformamid durchgeführt wird.

5. Verfahren zur Herstellung eines Medikamentes mit insbesondere anti-thrombotischen Wirkungen und Hypolipämie-Wirkung, dadurch gekennzeichnet, daß man eine Verbindung, wie sie in Anspruch 1 definiert wurde, verwendet.